# EUROPEAN PATENT APPLICATION

(11) **EP 1 974 750 A1**
(43) Date of publication of application: **01.10.2008**
(21) Application number: 07706682.7
(22) Date of filing: 12.01.2007
(51) Int. Cl.: A61K 45/06, A61K 9/127, A61K 31/7032, A61K 39/00, A61P 37/02, A61P 37/08, C07K 14/415, C07K 19/00

(54) **PREVENTIVE OR THERAPEUTIC AGENT AND METHOD FOR IMMUNE DISEASE**

(30) Priority: 13.01.2006 JP 2006005658
(71) Applicant: Riken, Wako-shi, Saitama 351-0198 (JP)
(72) Inventor: ISHII, Yasuyuki, RIKEN Yokohama Institute, Yokohama-shi, Kanagawa 2300045 (JP); NOZAWA, Risa, RIKEN Yokohama Institute, Yokohama-shi, Kanagawa 2300045 (JP); MATSUI, Yukiko, RIKEN Yokohama Institute, Yokohama-shi, Kanagawa 2300045 (JP)
(74) Representative: Hayes, Adrian Chetwynd
(86) International application number: PCT/JP2007/050340
(87) International publication number: WO 2007/080977

(57) **Abstract**

The present invention provides a method of selectively augmenting an immunosuppressive function in various functions of NKT cells, and a method of efficiently inducing an antigen specific immunosuppression *in vivo*, as well as a pharmaceutical obtained by applying the same.

More particularly, the present invention provides a pharmaceutical (e.g., a preventive or therapeutic agent for immune diseases such as allergic disease and autoimmune disease) containing a CD1d ligand and a target antigen (e.g., allergen, autoantigen). Preferably, the pharmaceutical contains a drug delivery vehicle including the CD1d ligand and the target antigen and having a lumen.

SEQUENCE LISTING

<110> RIKEN

<120> Prophylactic or therapeutic agents for immune diseases and methods using the same

<130> 091013

<150> JP 2006-5658
<151> 2006-1-13

<160> 11

<170> PatentIn version 3.3

<210> 1
<211> 24
<212> DNA
<213> Artificial

<220>
<223> primer

<400> 1
ccggtctaga aaagttgagc attc 24

<210> 2
<211> 21
<212> DNA
<213> Artificial

<220>
<223> primer

<400> 2
cctctgctct tgagttttcc c 21

<210> 3
<211> 21
<212> DNA
<213> Artificial

<220>
<223> primer

<400> 3
gggaaaactc aagagcagag g 21

<210> 4
<211> 31
<212> DNA
<213> Artificial

<220>
<223> primer

<400> 4
ccggaattcc tatcaacttg gacttaaatt c 31

<210> 5
<211> 17
<212> DNA
<213> Artificial

<220>
<223> primer

<400> 5
agaaaagttg agcattc 17

<210> 6
<211> 18
<212> DNA
<213> Artificial

<220>
<223> primer

<400> 6
tctgataatc ccatagac 18


<210> 7
<211> 37
<212> DNA
<213> Artificial

<220>
<223> primer

<400> 7
gaatgctcaa cttttctaca acgtttagag agagagc 37


<210> 8
<211> 2283
<212> DNA
<213> Artificial

<220>
<223> Synthetic Construct


<220>
<221> CDS
<222> (1)..(2283)

<400> 8
atg gca cat cac cac cac cat cac tcc gcg gct ctt gaa gtc ctc ttt 48
Met Ala His His His His His His Ser Ala Ala Leu Glu Val Leu Phe
1               5                   10                  15

cag gga ccc tct gat aat ccc ata gat agc tgc tgg aga gga gac tca 96
Gln Gly Pro Ser Asp Asn Pro Ile Asp Ser Cys Trp Arg Gly Asp Ser
            20                  25                  30

aac tgg gca caa aac aga atg aag ctc gca gat tgt gca gtg ggc ttc 144
Asn Trp Ala Gln Asn Arg Met Lys Leu Ala Asp Cys Ala Val Gly Phe
        35                  40                  45

gga agc tcc acc atg gga ggc aag gga gga gat ctt tat aca gtc aca 192
Gly Ser Ser Thr Met Gly Gly Lys Gly Gly Asp Leu Tyr Thr Val Thr
    50                  55                  60

aac tca gat gac gat cct gtg aat cct gca cca gga act ctg cgc tat 240
Asn Ser Asp Asp Asp Pro Val Asn Pro Ala Pro Gly Thr Leu Arg Tyr
65                  70                  75                  80

gga gca acc cga gat agg ccc ctg tgg ata att ttc agt ggg aat atg 288
Gly Ala Thr Arg Asp Arg Pro Leu Trp Ile Ile Phe Ser Gly Asn Met
                85                  90                  95

aat ata aag ctc aaa atg cct atg tac att gct ggg tat aag act ttt 336
Asn Ile Lys Leu Lys Met Pro Met Tyr Ile Ala Gly Tyr Lys Thr Phe
            100                 105                 110

gat ggc agg gga gca caa gtt tat att ggc aat ggc ggt ccc tgt gtg 384
Asp Gly Arg Gly Ala Gln Val Tyr Ile Gly Asn Gly Gly Pro Cys Val
        115                 120                 125

ttt atc aag aga gtg agc aat gtt atc ata cac ggt ttg cat ctg tac 432
Phe Ile Lys Arg Val Ser Asn Val Ile Ile His Gly Leu His Leu Tyr
    130                 135                 140

ggc tgt agt act agt gtt ttg ggg aat gtt ttg ata aac gag agt ttt 480
Gly Cys Ser Thr Ser Val Leu Gly Asn Val Leu Ile Asn Glu Ser Phe
145                 150                 155                 160

ggg gtg gag cct gtt cat cct cag gat ggc gat gct ctt act ctg cgc 528
Gly Val Glu Pro Val His Pro Gln Asp Gly Asp Ala Leu Thr Leu Arg
                165                 170                 175

act gct aca aat att tgg att gat cat aat tct ttc tcc aat tct tct 576
Thr Ala Thr Asn Ile Trp Ile Asp His Asn Ser Phe Ser Asn Ser Ser
            180                 185                 190

gat ggt ctg gtc gat gtc act ctt tct tcg act gga gtt act att tcc 624
Asp Gly Leu Val Asp Val Thr Leu Ser Ser Thr Gly Val Thr Ile Ser
        195                 200                 205

aac aat ctt ttt ttc aac cat cat aaa gtg atg ttg tta ggg cat gat 672
Asn Asn Leu Phe Phe Asn His His Lys Val Met Leu Leu Gly His Asp
    210                 215                 220

gat gca tat agt gat gac aaa tcc atg aag gtg aca gtg gcg ttc aat 720
Asp Ala Tyr Ser Asp Asp Lys Ser Met Lys Val Thr Val Ala Phe Asn
225                 230                 235                 240

caa ttt gga cct aac tgt gga caa aga atg ccc agg gca cga tat gga 768
Gln Phe Gly Pro Asn Cys Gly Gln Arg Met Pro Arg Ala Arg Tyr Gly
                245                 250                 255

ctt gta cat gtt gca aac aat aat tat gac cca tgg act ata tat gca 816
Leu Val His Val Ala Asn Asn Asn Tyr Asp Pro Trp Thr Ile Tyr Ala
            260                 265                 270

att ggt ggg agt tca aat cca acc att cta agt gaa ggg aat agt ttc 864
Ile Gly Gly Ser Ser Asn Pro Thr Ile Leu Ser Glu Gly Asn Ser Phe
        275                 280                 285

act gca cca aat gag agc tac aag aag caa gta acc ata cgt att gga 912
Thr Ala Pro Asn Glu Ser Tyr Lys Lys Gln Val Thr Ile Arg Ile Gly
    290                 295                 300

tgc aaa aca tca tca tct tgt tca aat tgg gtg tgg caa tct aca caa 960
Cys Lys Thr Ser Ser Ser Cys Ser Asn Trp Val Trp Gln Ser Thr Gln
305                 310                 315                 320

gat gtt ttt tat aat gga gct tat ttt gta tca tca ggg aaa tat gaa 1008
Asp Val Phe Tyr Asn Gly Ala Tyr Phe Val Ser Ser Gly Lys Tyr Glu
                325                 330                 335

ggg ggt aat ata tac aca aag aaa gaa gct ttc aat gtt gag aat ggg 1056
Gly Gly Asn Ile Tyr Thr Lys Lys Glu Ala Phe Asn Val Glu Asn Gly
            340                 345                 350

aat gca act cct caa ttg aca aaa aat gct ggg gtt tta aca tgc tct 1104
Asn Ala Thr Pro Gln Leu Thr Lys Asn Ala Gly Val Leu Thr Cys Ser
        355                 360                 365

ctc tct aaa cgt tgt aga aaa gtt gag cat tct cgt cat gat gct atc 1152
Leu Ser Lys Arg Cys Arg Lys Val Glu His Ser Arg His Asp Ala Ile
    370                 375                 380

aac atc ttc aat gtg gaa aaa tat ggc gca gta ggc gat gga aag cat 1200
Asn Ile Phe Asn Val Glu Lys Tyr Gly Ala Val Gly Asp Gly Lys His
385                 390                 395                 400

gat tgc act gag gca ttt tca aca gca tgg caa gct gca tgc aaa aag 1248
Asp Cys Thr Glu Ala Phe Ser Thr Ala Trp Gln Ala Ala Cys Lys Lys
                405                 410                 415

cca tca gca atg ttg ctt gtg cca ggc aac aag aaa ttt gtt gta aac 1296
Pro Ser Ala Met Leu Leu Val Pro Gly Asn Lys Lys Phe Val Val Asn
            420                 425                 430

aat ttg ttc ttc aat ggg cca tgt caa cct cac ttt act ttt aag gta 1344
Asn Leu Phe Phe Asn Gly Pro Cys Gln Pro His Phe Thr Phe Lys Val
        435                 440                 445

gat ggg ata ata gct gcg tac caa aat cca gct agc tgg aag aat aat 1392
Asp Gly Ile Ile Ala Ala Tyr Gln Asn Pro Ala Ser Trp Lys Asn Asn
    450                 455                 460

aga ata tgg ttg cag ttt gct aaa ctt aca ggt ttt act cta atg ggt 1440
Arg Ile Trp Leu Gln Phe Ala Lys Leu Thr Gly Phe Thr Leu Met Gly
465                 470                 475                 480

aaa ggt gta att gat ggg caa gga aaa caa tgg tgg gct ggc caa tgt 1488
Lys Gly Val Ile Asp Gly Gln Gly Lys Gln Trp Trp Ala Gly Gln Cys
                485                 490                 495

aaa tgg gtc aat gga cga gaa att tgc aac gat cgt gat aga cca aca 1536
Lys Trp Val Asn Gly Arg Glu Ile Cys Asn Asp Arg Asp Arg Pro Thr
            500                 505                 510

gcc att aaa ttc gat ttt tcc acg ggt ctg ata atc caa gga ctg aaa 1584
Ala Ile Lys Phe Asp Phe Ser Thr Gly Leu Ile Ile Gln Gly Leu Lys
        515                 520                 525

cta atg aac agc ccc gaa ttt cat tta gtt ttt ggg aat tgt gag gga 1632
Leu Met Asn Ser Pro Glu Phe His Leu Val Phe Gly Asn Cys Glu Gly
    530                 535                 540

gta aaa atc atc ggc att agt att acg gca ccg aga gac agt cct aac 1680
Val Lys Ile Ile Gly Ile Ser Ile Thr Ala Pro Arg Asp Ser Pro Asn
545                 550                 555                 560

act gat gga att gat atc ttt gca tct aaa aac ttt cac tta caa aag 1728
Thr Asp Gly Ile Asp Ile Phe Ala Ser Lys Asn Phe His Leu Gln Lys
                565                 570                 575

aac acg ata gga aca ggg gat gac tgc gtc gct ata ggc aca ggg tct 1776
Asn Thr Ile Gly Thr Gly Asp Asp Cys Val Ala Ile Gly Thr Gly Ser
            580                 585                 590

tct aat att gtg att gag gat ctg att tgc ggt cca ggc cat gga ata 1824
Ser Asn Ile Val Ile Glu Asp Leu Ile Cys Gly Pro Gly His Gly Ile
        595                 600                 605

agt ata gga agt ctt ggg agg gaa aac tct aga gca gag gtt tca tac 1872
Ser Ile Gly Ser Leu Gly Arg Glu Asn Ser Arg Ala Glu Val Ser Tyr
    610                 615                 620

gtg cac gta aat ggg gct aaa ttc ata gac aca caa aat gga tta aga 1920
Val His Val Asn Gly Ala Lys Phe Ile Asp Thr Gln Asn Gly Leu Arg
625                 630                 635                 640

atc aaa aca tgg cag ggt ggt tca ggc atg gca agc cat ata att tat 1968
Ile Lys Thr Trp Gln Gly Gly Ser Gly Met Ala Ser His Ile Ile Tyr
                645                 650                 655

gag aat gtt gaa atg ata aat tcg gag aac ccc ata tta ata aat caa 2016
Glu Asn Val Glu Met Ile Asn Ser Glu Asn Pro Ile Leu Ile Asn Gln
            660                 665                 670

ttc tac tgc act tcg gct tct gct tgc caa aac cag agg tct gcg gtt 2064
Phe Tyr Cys Thr Ser Ala Ser Ala Cys Gln Asn Gln Arg Ser Ala Val
        675                 680                 685

caa atc caa gat gtg aca tac aag aac ata cgt ggg aca tca gca aca 2112
Gln Ile Gln Asp Val Thr Tyr Lys Asn Ile Arg Gly Thr Ser Ala Thr
    690                 695                 700

gca gca gca att caa ctt aag tgc agt gac agt atg ccc tgc aaa gat 2160
Ala Ala Ala Ile Gln Leu Lys Cys Ser Asp Ser Met Pro Cys Lys Asp
705                 710                 715                 720

ata aag cta agt gat ata tct ttg aag ctt acc tca ggg aaa att gct 2208
Ile Lys Leu Ser Asp Ile Ser Leu Lys Leu Thr Ser Gly Lys Ile Ala
                725                 730                 735

tcc tgc ctt aat gat aat gca aat gga tat ttc agt gga cac gtc atc 2256
Ser Cys Leu Asn Asp Asn Ala Asn Gly Tyr Phe Ser Gly His Val Ile

740                 745                 750

cct gca tgc aag aat tta agt cca agt                                    2283
Pro Ala Cys Lys Asn Leu Ser Pro Ser
        755                 760

<210>  9
<211>  761
<212>  PRT
<213>  Artificial

<220>
<223>  Synthetic Construct

<400>  9

Met Ala His His His His His His Ser Ala Ala Leu Glu Val Leu Phe
1               5                   10                  15

Gln Gly Pro Ser Asp Asn Pro Ile Asp Ser Cys Trp Arg Gly Asp Ser
            20                  25                  30

Asn Trp Ala Gln Asn Arg Met Lys Leu Ala Asp Cys Ala Val Gly Phe
        35                  40                  45

Gly Ser Ser Thr Met Gly Gly Lys Gly Gly Asp Leu Tyr Thr Val Thr
    50                  55                  60

Asn Ser Asp Asp Asp Pro Val Asn Pro Ala Pro Gly Thr Leu Arg Tyr
65                  70                  75                  80

Gly Ala Thr Arg Asp Arg Pro Leu Trp Ile Ile Phe Ser Gly Asn Met
                85                  90                  95

Asn Ile Lys Leu Lys Met Pro Met Tyr Ile Ala Gly Tyr Lys Thr Phe
            100                 105                 110

Asp Gly Arg Gly Ala Gln Val Tyr Ile Gly Asn Gly Gly Pro Cys Val
        115                 120                 125

Phe Ile Lys Arg Val Ser Asn Val Ile Ile His Gly Leu His Leu Tyr
    130                 135                 140

Gly Cys Ser Thr Ser Val Leu Gly Asn Val Leu Ile Asn Glu Ser Phe
145                 150                 155                 160

Gly Val Glu Pro Val His Pro Gln Asp Gly Asp Ala Leu Thr Leu Arg
                165                 170                 175

Thr Ala Thr Asn Ile Trp Ile Asp His Asn Ser Phe Ser Asn Ser Ser
            180                 185                 190

Asp Gly Leu Val Asp Val Thr Leu Ser Ser Thr Gly Val Thr Ile Ser
        195                 200                 205

Asn Asn Leu Phe Phe Asn His His Lys Val Met Leu Leu Gly His Asp
    210                 215                 220

Asp Ala Tyr Ser Asp Asp Lys Ser Met Lys Val Thr Val Ala Phe Asn
225                 230                 235                 240

Gln Phe Gly Pro Asn Cys Gly Gln Arg Met Pro Arg Ala Arg Tyr Gly
                245                 250                 255

Leu Val His Val Ala Asn Asn Asn Tyr Asp Pro Trp Thr Ile Tyr Ala
            260                 265                 270

Ile Gly Gly Ser Ser Asn Pro Thr Ile Leu Ser Glu Gly Asn Ser Phe
        275                 280                 285

Thr Ala Pro Asn Glu Ser Tyr Lys Lys Gln Val Thr Ile Arg Ile Gly
    290                 295                 300

Cys Lys Thr Ser Ser Ser Cys Ser Asn Trp Val Trp Gln Ser Thr Gln
305                 310                 315                 320

Asp Val Phe Tyr Asn Gly Ala Tyr Phe Val Ser Ser Gly Lys Tyr Glu
                325                 330                 335

Gly Gly Asn Ile Tyr Thr Lys Lys Glu Ala Phe Asn Val Glu Asn Gly
            340                 345                 350

Asn Ala Thr Pro Gln Leu Thr Lys Asn Ala Gly Val Leu Thr Cys Ser
        355                 360                 365

Leu Ser Lys Arg Cys Arg Lys Val Glu His Ser Arg His Asp Ala Ile
    370                 375                 380

Asn Ile Phe Asn Val Glu Lys Tyr Gly Ala Val Gly Asp Gly Lys His
385                 390                 395                 400

Asp Cys Thr Glu Ala Phe Ser Thr Ala Trp Gln Ala Ala Cys Lys Lys
                405                 410                 415

Pro Ser Ala Met Leu Leu Val Pro Gly Asn Lys Lys Phe Val Val Asn
            420                 425                 430

Asn Leu Phe Phe Asn Gly Pro Cys Gln Pro His Phe Thr Phe Lys Val
        435                 440                 445

Asp Gly Ile Ile Ala Ala Tyr Gln Asn Pro Ala Ser Trp Lys Asn Asn
    450                 455                 460

Arg Ile Trp Leu Gln Phe Ala Lys Leu Thr Gly Phe Thr Leu Met Gly
465                 470                 475                 480

Lys Gly Val Ile Asp Gly Gln Gly Lys Gln Trp Trp Ala Gly Gln Cys
                485                 490                 495

Lys Trp Val Asn Gly Arg Glu Ile Cys Asn Asp Arg Asp Arg Pro Thr
            500                 505                 510

Ala Ile Lys Phe Asp Phe Ser Thr Gly Leu Ile Ile Gln Gly Leu Lys
        515                 520                 525

Leu Met Asn Ser Pro Glu Phe His Leu Val Phe Gly Asn Cys Glu Gly

530                 535                 540

Val Lys Ile Ile Gly Ile Ser Ile Thr Ala Pro Arg Asp Ser Pro Asn
545                 550                 555                 560

Thr Asp Gly Ile Asp Ile Phe Ala Ser Lys Asn Phe His Leu Gln Lys
                565                 570                 575

Asn Thr Ile Gly Thr Gly Asp Asp Cys Val Ala Ile Gly Thr Gly Ser
            580                 585                 590

Ser Asn Ile Val Ile Glu Asp Leu Ile Cys Gly Pro Gly His Gly Ile
        595                 600                 605

Ser Ile Gly Ser Leu Gly Arg Glu Asn Ser Arg Ala Glu Val Ser Tyr
    610                 615                 620

Val His Val Asn Gly Ala Lys Phe Ile Asp Thr Gln Asn Gly Leu Arg
625                 630                 635                 640

Ile Lys Thr Trp Gln Gly Gly Ser Gly Met Ala Ser His Ile Ile Tyr
                645                 650                 655

Glu Asn Val Glu Met Ile Asn Ser Glu Asn Pro Ile Leu Ile Asn Gln
            660                 665                 670

Phe Tyr Cys Thr Ser Ala Ser Ala Cys Gln Asn Gln Arg Ser Ala Val
        675                 680                 685

Gln Ile Gln Asp Val Thr Tyr Lys Asn Ile Arg Gly Thr Ser Ala Thr
    690                 695                 700

Ala Ala Ala Ile Gln Leu Lys Cys Ser Asp Ser Met Pro Cys Lys Asp
705                 710                 715                 720

Ile Lys Leu Ser Asp Ile Ser Leu Lys Leu Thr Ser Gly Lys Ile Ala
                725                 730                 735

Ser Cys Leu Asn Asp Asn Ala Asn Gly Tyr Phe Ser Gly His Val Ile
            740                 745                 750

Pro Ala Cys Lys Asn Leu Ser Pro Ser
        755                 760

<210>  10
<211>  354
<212>  PRT
<213>  Cryptomeria japonica

<400>  10

Ser Asp Asn Pro Ile Asp Ser Cys Trp Arg Gly Asp Ser Asn Trp Ala
1               5                   10                  15

Gln Asn Arg Met Lys Leu Ala Asp Cys Ala Val Gly Phe Gly Ser Ser
            20                  25                  30

Thr Met Gly Gly Lys Gly Gly Asp Leu Tyr Thr Val Thr Asn Ser Asp

        35                  40                  45

Asp Asp Pro Val Asn Pro Ala Pro Gly Thr Leu Arg Tyr Gly Ala Thr
    50                  55                  60

Arg Asp Arg Pro Leu Trp Ile Ile Phe Ser Gly Asn Met Asn Ile Lys
65                  70                  75                  80

Leu Lys Met Pro Met Tyr Ile Ala Gly Tyr Lys Thr Phe Asp Gly Arg
                85                  90                  95

Gly Ala Gln Val Tyr Ile Gly Asn Gly Gly Pro Cys Val Phe Ile Lys
            100                 105                 110

Arg Val Ser Asn Val Ile Ile His Gly Leu His Leu Tyr Gly Cys Ser
        115                 120                 125

Thr Ser Val Leu Gly Asn Val Leu Ile Asn Glu Ser Phe Gly Val Glu
    130                 135                 140

Pro Val His Pro Gln Asp Gly Asp Ala Leu Thr Leu Arg Thr Ala Thr
145                 150                 155                 160

Asn Ile Trp Ile Asp His Asn Ser Phe Ser Asn Ser Ser Asp Gly Leu
                165                 170                 175

Val Asp Val Thr Leu Ser Ser Thr Gly Val Thr Ile Ser Asn Asn Leu
            180                 185                 190

Phe Phe Asn His His Lys Val Met Leu Leu Gly His Asp Asp Ala Tyr
        195                 200                 205

Ser Asp Asp Lys Ser Met Lys Val Thr Val Ala Phe Asn Gln Phe Gly
    210                 215                 220

Pro Asn Cys Gly Gln Arg Met Pro Arg Ala Arg Tyr Gly Leu Val His
225                 230                 235                 240

Val Ala Asn Asn Asn Tyr Asp Pro Trp Thr Ile Tyr Ala Ile Gly Gly
                245                 250                 255

Ser Ser Asn Pro Thr Ile Leu Ser Glu Gly Asn Ser Phe Thr Ala Pro
            260                 265                 270

Asn Glu Ser Tyr Lys Lys Gln Val Thr Ile Arg Ile Gly Cys Lys Thr
        275                 280                 285

Ser Ser Ser Cys Ser Asn Trp Val Trp Gln Ser Thr Gln Asp Val Phe
    290                 295                 300

Tyr Asn Gly Ala Tyr Phe Val Ser Ser Gly Lys Tyr Glu Gly Gly Asn
305                 310                 315                 320

Ile Tyr Thr Lys Lys Glu Ala Phe Asn Val Glu Asn Gly Asn Ala Thr
                325                 330                 335

Pro Gln Leu Thr Lys Asn Ala Gly Val Leu Thr Cys Ser Leu Ser Lys
            340                 345                 350

Arg Cys

<210> 11
<211> 388
<212> PRT
<213> Cryptomeria japonica

<400> 11

Arg Lys Val Glu His Ser Arg His Asp Ala Ile Asn Ile Phe Asn Val
1               5                   10                  15

Glu Lys Tyr Gly Ala Val Gly Asp Gly Lys His Asp Cys Thr Glu Ala
            20                  25                  30

Phe Ser Thr Ala Trp Gln Ala Ala Cys Lys Lys Pro Ser Ala Met Leu
        35                  40                  45

Leu Val Pro Gly Asn Lys Lys Phe Val Val Asn Asn Leu Phe Phe Asn
    50                  55                  60

Gly Pro Cys Gln Pro His Phe Thr Phe Lys Val Asp Gly Ile Ile Ala
65                  70                  75                  80

Ala Tyr Gln Asn Pro Ala Ser Trp Lys Asn Asn Arg Ile Trp Leu Gln
                85                  90                  95

Phe Ala Lys Leu Thr Gly Phe Thr Leu Met Gly Lys Gly Val Ile Asp
            100                 105                 110

Gly Gln Gly Lys Gln Trp Trp Ala Gly Gln Cys Lys Trp Val Asn Gly
        115                 120                 125

Arg Glu Ile Cys Asn Asp Arg Asp Arg Pro Thr Ala Ile Lys Phe Asp
    130                 135                 140

Phe Ser Thr Gly Leu Ile Ile Gln Gly Leu Lys Leu Met Asn Ser Pro
145                 150                 155                 160

Glu Phe His Leu Val Phe Gly Asn Cys Glu Gly Val Lys Ile Ile Gly
                165                 170                 175

Ile Ser Ile Thr Ala Pro Arg Asp Ser Pro Asn Thr Asp Gly Ile Asp
            180                 185                 190

Ile Phe Ala Ser Lys Asn Phe His Leu Gln Lys Asn Thr Ile Gly Thr
        195                 200                 205

Gly Asp Asp Cys Val Ala Ile Gly Thr Gly Ser Ser Asn Ile Val Ile
    210                 215                 220

Glu Asp Leu Ile Cys Gly Pro Gly His Gly Ile Ser Ile Gly Ser Leu
225                 230                 235                 240

Gly Arg Glu Asn Ser Arg Ala Glu Val Ser Tyr Val His Val Asn Gly
                245                 250                 255

Ala Lys Phe Ile Asp Thr Gln Asn Gly Leu Arg Ile Lys Thr Trp Gln
            260                 265                 270

Gly Gly Ser Gly Met Ala Ser His Ile Ile Tyr Glu Asn Val Glu Met
        275                 280                 285

Ile Asn Ser Glu Asn Pro Ile Leu Ile Asn Gln Phe Tyr Cys Thr Ser
    290                 295                 300

Ala Ser Ala Cys Gln Asn Gln Arg Ser Ala Val Gln Ile Gln Asp Val
305                 310                 315                 320

Thr Tyr Lys Asn Ile Arg Gly Thr Ser Ala Thr Ala Ala Ala Ile Gln
                325                 330                 335

Leu Lys Cys Ser Asp Ser Met Pro Cys Lys Asp Ile Lys Leu Ser Asp
            340                 345                 350

Ile Ser Leu Lys Leu Thr Ser Gly Lys Ile Ala Ser Cys Leu Asn Asp
        355                 360                 365

Asn Ala Asn Gly Tyr Phe Ser Gly His Val Ile Pro Ala Cys Lys Asn
    370                 375                 380

Leu Ser Pro Ser
385
t

## Description

### Technical Field

The present invention relates to a preventive or therapeutic agent for an immune disease such as an allergic disease or an autoimmune disease, a preventive or therapeutic method for the immune disease, and a cedar pollen antigen-fusing protein.

### Background Art

It has been desired earnestly to develop a method capable of fundamentally treating immune diseases including autoimmune diseases and allergic diseases caused by abnormality in immune response by taking advantage of immunosuppressive mechanisms because many of current therapeutic methods for these diseases are symptomatic therapies. As major cell populations involved in immunoregulatory mechanisms, NKT cells , tolerance inducible dendritic cells and regulatory T cells have been already reported. Therefore, a method of taking advantage of the immunoregulatory mechanism through these immune cells is thought as the method capable of fundamentally treating the immune disease.

The following reports have been available as the methods of taking advantage of the immunoregulatory mechanism, which can lead to the treatment of the immune disease.

Patent document 1 discloses a method of enclosing an OVA (ovalbumin) protein in a liposome lumen including α-GalCer (α-galactosylceramide) and a suppressive effect by the above liposome on antibody production in mice.

In Non-patent literature 1, it has been described that when murine bone marrow cells are cultured *in vitro* in the presence of IL-10, CD45RB^{high} CD11c^{low} cells proliferate, and that the CD45RB^{high} CD11c^{low} cells are present in spleen and regulatory T cells can be differentiated and proliferated from naive CD4⁺ T cells *in vitro* and *in vivo* in mice.

In Non-patent literature 2, a method for artificially producing regulatory dendritic cells from the murine bone marrow cells has been described.

In Non-patent literature 3, it has been described that low density B220 positive B cells in murine spleen have a capacity to produce IL-10 by stimulating with bacteria.

In Non-patent literature 4, it has been described that low density B cells in spleen in the mouse administered with α-GalCer can not enhance the capacity of NKT cells to produce IL-4 and they suppress the capacity of DC-activated NKT cells to produce IFN-γ and IL-4.

However, a method of selectively augmenting an immunosuppressive function from various functions of the NKT cells and a method of efficiently inducing antigen specific immunosuppression *in vivo* have not been developed yet.
Patent document 1: International Publication WO2005/120574 Pamphlet
Non-patent literature 1: Wakkach et al., Immunity 18: 605-617 (2003)
Non-patent literature 2: Sato et al., Immunity 18: 367-379 (2003)
Non-patent literature 3: Burke et al., The Journal of Immunology 173: 2362-2372 (2004)
Non-patent literature 4: Bezbradica et al., The Journal of Immunology 174: 4696-4705 (2005)

### DISCLOSURE OF THE INVENTION

### PROBLEMS TO BE SOLVED BY THE INVENTION

Therefore, it is an object of the present invention to provide a method of selectively augmenting an immunosuppressive function in various functions of NKT cells and a method of efficiently inducing antigen specific immunosuppression *in vivo* as well as a pharmaceutical obtained by applying the same.

### MEANS FOR SOLVING THE PROBLEMS

As a result of an extensive study, the present inventors have found that since a drug delivery vehicle comprising CD1d ligand and a natural or recombinant allergen specifically inhibits IgE production caused by the allergen, allergic diseases caused by the allergen can be specifically treated with such a drug delivery vehicle. Furthermore, they have conceived from such a finding that autoimmune diseases caused by an autoantigen can be likewise treated specifically with a drug delivery vehicle comprising the autoantigen instead of the allergen, and thus, completed the present invention.

That is, the present invention provides the following inventions and the like.
[1] A preventive or therapeutic agent for an immune disease caused by a target antigen, containing a drug delivery vehicle comprising a CD1d ligand and the target antigen and having a lumen.
[2] The agent of [1] above wherein the target antigen is an allergen and the immune disease is an allergic disease caused by the allergen.
[3] The agent of [1] above wherein the drug delivery vehicle is a liposome.
[4] The agent of [1] above wherein the CD1d ligand is α-GalCer.
[5] The agent of [2] above wherein the allergen is a cedar pollen.
[6] The agent of [2] above wherein the allergen is a fusion protein of a Cryj1 mature protein and a Cryj2 mature protein.
[7] The agent of [5] above wherein the Cryj1 mature protein is present in the N terminal side of the Cryj2 mature protein in the fusion protein.
[8] The agent of [2] above wherein the CD1d ligand is embedded in a liposome membrane and the allergen is enclosed in a liposome lumen.
[9] The agent of [1] above wherein the target antigen is an autoantigen.
[10] A fusion protein comprising cedar pollen antigens, a Cryj1 protein and a Cryj2 protein.
[11] The fusion protein of [10] above wherein the Cryj1 protein is a Cryj1 mature protein and the Cryj2 protein is a Cryj2 mature protein.
[12] The fusion protein of [10] above wherein the Cryj1 protein and the Cryj2 protein are linked directly or via a peptide linker.
[13] The fusion protein of [10] above wherein the Cryj1 protein is present in its N terminal side and the Cryj2 mature protein is present in its C terminal side.
[14] A method for preventing or treating an immune disease comprising a step of administering a therapeutically effective amount of the preventive or therapeutic agent for the immune disease of any of [1] to [9] above to a subject in need of such a treatment.
[15] The method for preventing or treating the immune disease of [14] above wherein the subject is a human patient with the immune disease.

### BRIEF DESCRIPTION OF THE DRAWINGS

FIG. 1 is a view showing the production of IFN-γ, IL-4 and IL-10 in dendritic cells (DC) and B cells derived from spleen in mice administered with α-GalCer or αGC(α-GalCer)-liposome. "ND": not detected.
FIG. 2A is a view showing analysis of low density (LD) B cells and high density (HD) B cells by a flow cytometer.
FIG. 2B is a view showing amounts of IL-10 secreted in culture media by co-culturing the low density (LD) B cells or the high density (HD) B cells with whole spleen cells.
FIG. 3 is a view showing IL-10 production by marginal zone B cells pulsed with αGC-liposome. "ND": not detected.
FIG. 4 is a view showing IgE concentrations specific for anti-Cryj1 in blood in mice sensitized with natural type Cryj1 and administered with αGC-natural type Cryj1-liposome, αGC-liposome or liposome alone.

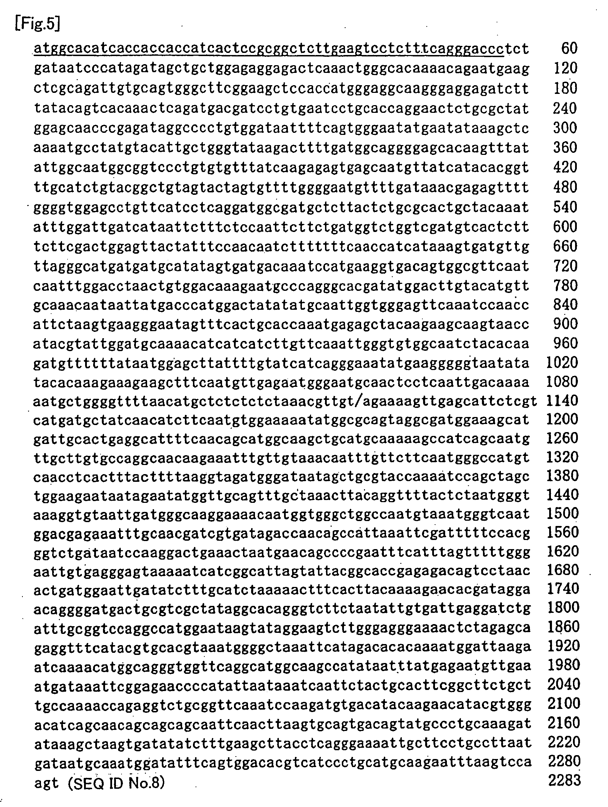

FIG. 5 is a view showing a nucleotide sequence (SEQ ID NO:8) of a Cryj1/2 gene. In the nucleotide sequence represented by SEQ ID NO:8, the nucleotide sequence composed of 1st to 57th nucleotide residues (underlined) is the nucleotide sequence of a His tag region derived from pET47b vector; the nucleotide sequence composed of 58th to 1119th nucleotide residues is the nucleotide sequence encoding an amino acid sequence of the Cryj1 mature protein (the amino acid sequence, SEQ ID NO:10 composed of 21st to 374th amino acid residues in the amino acid sequence registered as GenBank accession number BAA07020); and the nucleotide sequence composed of 1120th to 2283rd nucleotide residues is the nucleotide sequence encoding the amino acid sequence of the Cryj2 mature protein (the amino acid sequence, SEQ ID NO:11 composed of 46th to 433rd amino acid residues in the amino acid sequence registered as GenBank accession number P43212).

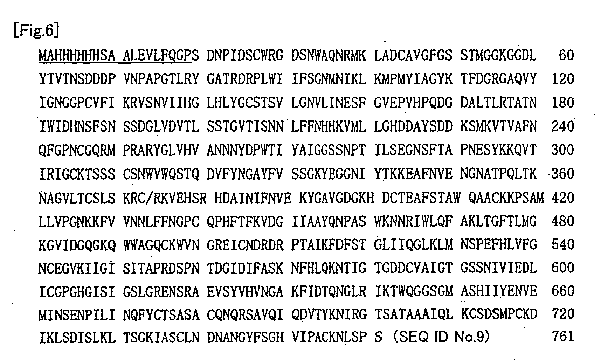

FIG. 6 is a view showing the amino acid sequence (SEQ ID NO:9) of the Cryj1/2 protein. In the amino acid sequence represented by SEQ ID NO:9, the amino acid sequence (underlined) composed of the 1st to 19th amino acid residues is the amino acid sequence of the His tag region derived from pET47b vector; the amino acid sequence composed of the 20th to 373rd amino acid residues is the amino acid sequence of the Cryj1 mature protein (the amino acid sequence, SEQ ID NO:10 composed of the 21st to 374th amino acid residues in the amino acid sequence registered as GenBank accession number BAA07020); and the amino acid sequence composed of the 374th to 761st amino acid residues is the amino acid sequence of the Cryj2 mature protein (the amino acid sequence, SEQ ID NO:11 composed of 46th to 433rd amino acid residues in the amino acid sequence registered as GenBank accession number P43212).
FIG. 7 is a view showing IgE antibody titers specific for the natural type Cryj1 in sera from mice immunized with the natural type Cryj1 protein or a recCryj1/2 protein. *: equal to or lower than a detection limit.
FIG. 8 is a view showing IgE antibody titers specific for the recCryj1/2 in sera from mice immunized with the natural type Cryj1 protein or the recCryj1/2 protein.
FIG. 9 is a view showing IgG antibody titers specific for the natural type Cryj1 in sera from mice immunized with the natural type Cryj1 protein or the recCryj1/2 protein.
FIG. 10 is a view showing suppression of increase of IgE antibody titers specific for the natural type Cryj1 by the recCryj1/2 protein in sera from mice immunized with the natural type Cryj1.
FIG. 11 is a view showing anti-Cryj1 IgE concentrations in blood from mice sensitized with the natural type Cryj1 and administered with αGC-recombinant Cryj1/2 fusion protein-liposome, αGC-liposome or saline.

### DETAILED DESCRIPTION OF PREFFERRED EMBODIMENT

In one embodiment, the present invention provides a preventive or therapeutic agent for an immune disease, containing a drug delivery vehicle (hereinafter if necessary referred to as the drug delivery vehicle, the drug delivery vehicle having a lumen, the drug delivery vehicle comprising a CD1d ligand or the drug delivery vehicle comprising a target antibody) comprising the CD1d ligand and the target antigen and having the lumen.

The drug delivery vehicle used in the present invention is not particularly limited as long as it enables to deliver the drug to an animal and has the lumen, and includes, for example, a liposome and a microsphere.

The liposome refers to a vesicle structure obtained by closing a micelle (water-soluble particles obtained by aggregating amphipathic molecules having a hydrophilic region and a hydrophobic region). When a pharmaceutical of the present invention comprises the liposome as the drug delivery vehicle, the CD1d ligand can be embedded in a liposome membrane and an allergen can be enclosed in a liposome lumen. When the liposome is used as the drug delivery vehicle, such a liposome can be produced by a method described in International Publication WO2005/120574. In detail, the liposome comprising the CD1d ligand such as α-GalCer can be obtained by mixing a lipid which composes the liposome with an organic solvent solution comprising the CD1d ligand followed by drying, then adding water thereto and giving an ultrasonic treatment, in accordance with standard methods as described in PCT/JP 2005/10254. The liposome enclosing the target antigen can be obtained by mixing the lipid which composes the liposome with the organic solvent solution comprising the CD1d ligand followed by drying, then adding an aqueous solution of the target antigen thereto and giving the ultrasonic treatment.

The microsphere refers to a fine spherical substance using an *in vivo* degradable polymer as a base. The microsphere includes, for example, a porous type microsphere and a capsule type microsphere.

The CD1d ligand refers to a substance presented on a CD1d expressing antigen presenting cell (APC) and thus capable of activating an NKT cell. The CD1d ligand includes, for example, α-GalCer and derivatives thereof as well as IGb3 (Isogloboglycosphingolipid) present *in vivo*, and α-GalCer is preferable.

The target antigen is not particularly limited as long as the inhibition of an immune response to the antigen is desired *in vivo*. The target antigen may be a natural antigen, a recombinant protein or a chemically synthesized coumpound, and includes, for example, an allergen, an autoantigen, and a graft alloantigen.
The pharmaceutical of the present invention will be described in detail below for the case of applying to allergic diseases and autoimmune diseases.

### [Application to allergic diseases]

The allergen is not particularly limited as long as it is a factor capable of causing the allergy when exposed, ingested or applied *in vivo*. Such an allergen includes, for example factors capable of causing the allergy, contained in pollens (e.g., of cedar, Japanese cypress, ragweed, rice, Betula, cocksfoot and tansy), foods (e.g., cow milks, buckwheat noodles, eggs, peanuts, wheat, soybeans, fish and shellfish, fruits or processed foods thereof), organisms other than human beings or materials derived therefrom (e.g., mite, fungus, body hairs of animals or birds, bee toxin), chemicals (e.g., penicillin-based antibiotics, sulfa drugs, barbiturate derivatives), medical supplies (e.g., natural rubber gloves), livingwares (e.g., metals of accessories), other substances or compositions (e.g., latex).

The pharmaceutical of the present invention is useful as the therapeutic agent specific for the allergic disease caused by an allergen when comprising the drug deliver vehicle comprising the allergen as the target antigen. The present inventors have found that since the drug delivery vehicle comprising the CD1d ligand and the allergen specifically inhibits the IgE production caused by the allergen, the allergic disease caused by the allergen can be specifically treated with such a drug delivery vehicle. The allergic diseases capable of being specifically treated with the drug delivery vehicle of the present invention include, for example, atopic bronchial asthma, atopic dermatitis, allergic rhinitis (e.g., pollen disease), allergic conjunctivitis, food allergy and drug allergy.

Preferable examples as the allergen can be cedar pollen antigens (e.g., proteins such as Cryj1 and Cryj2). A recombinant fusion protein of the cedar pollen antigens is also preferable as the allergen. Such a fusion protein includes, for example, a fusion protein of the Cryj1 protein and the Cryj2 protein (hereinafter referred to as the "fusion protein" as needed).

The Cryj1 mature protein is a polypeptide obtained by at least partially removing a signal region present in the N terminal side in a polypeptide expressed by a Cryj1 gene. For example, with reference to GenBank accession number BAA07020, the polypeptide composed of 374 amino acid residues has been registered as the Cryj1 protein. The Cryj1 mature protein corresponds to the polypeptide composed of the 22nd to 374th amino acid residues in the polypeptide composed of the 374 amino acid residues registered as BAA07020. The region composed of the 1st to 21st amino acid residues in the polypeptide composed of the 374 amino acid residues registered as BAA07020 is the signal region.

The Cryj1 mature protein can be a natural Cryj1 mature protein or a mutant protein having one or more (e.g., 1 to 10, preferably 1 to 7, more preferably 1 to 5 and most preferably 1, 2 or 3) modifications (e.g., substitution, addition, insertion, deletion) in the natural Cryj1 mature protein, or having at least about 95%, preferably about 97%, more preferably about 98% and most preferably about 99% amino acid sequence identity to the amino acid sequence of the natural Cryj1 mature protein, and keeping an epitope in the natural Cryj1 mature protein. The natural Cryj1 mature protein includes the polypeptide composed of the 22nd to 374th amino acid residue in the polypeptide composed of the 374 amino acid residue registered as GenBank accession No. BAA07020, and naturally occurring isotypes thereof (e.g., see GenBank accession numbers D34639, D26544, D26545, AB081309 and AB081310). The mutant protein can be the protein modified to keep one or two or more, preferably all epitopes (e.g., T cell epitopes and B cell epitopes) in the Cryj1 mature protein.

The Cryj2 mature protein is the polypeptide obtained by removing the signal region present in the N terminal side and two pro regions present at the N terminus and C terminus, respectively of the coding region of the mature protein. For example, with reference to GenBank accession number P43212, the polypeptide composed of 514 amino acid residues has been registered as the Cryj2 protein. The Cryj2 mature protein corresponds to the polypeptide composed of the 46th to 433rd amino acid residues in the polypeptide composed of 514 amino acid residues registered as GenBank accession No.P43212. In the polypeptide composed of 514 amino acid residues registered as P43212, the region composed of the 1st to 22nd amino acid residues is the signal region, the region composed of the 23rd to 45th amino acid residues and the region composed of 434th to 514th amino acid residues are the pro regions.

The Cryj2 mature protein can be a natural Cryj2 mature protein or a mutant protein having one or more (e.g., 1 to 10, preferably 1 to 7, more preferably 1 to 5 and most preferably 1, 2 or 3) modifications (e.g., substitution, addition, insertion, deletion) in the natural Cryj2 mature protein, or having at least about 95%, preferably about 97%, more preferably about 98% and most preferably about 99% amino acid sequence identity to the amino acid sequence of the natural Cryj2 mature protein, and keeping an epitope in the natural Cryj2 mature protein. The natural Cryj2 mature protein includes the polypeptide composed of the 46th to 433rd amino acid residues in the polypeptide composed of 514 amino acid residues registered as P43212, and naturally occurring isotypes thereof (e.g., see GenBank accession numbers D37765, D29772, E10716, AB081403, AB081404 and AB081405). The mutant protein can be the protein modified to keep one or two or more, preferably all epitopes (e.g., T cell epitopes and B cell epitopes) in the Cryj2 mature protein.

The amino acid sequence identity (%) can be determined using a program (e.g., BLAST, FASTA) used commonly in the art in default configuration. The identity (%) can also be determined using an optional algorithm known publicly, e.g., the algorithm of Needleman et al., (1970) (J. Mol. Biol., 48: 444-453), or Myers and Miller (CABIOS, 1988, 4: 11-17). The algorithm of Needleman et al. is incorporated in GAP program in GCG software package (available at www.gcg.com), and the identity (%) can also be determined using, for example, any of BLOSUM 62 matrix or PAM250 matrix, as well as gap weight: 16, 14, 12, 10, 8, 6 or 4, and length weight: 1, 2, 3, 4, 5 or 6. The algorithm of Myers and Miller is incorporated in ALIGN program which is a part of GCG sequence alignment software package. When the ALIGN program is used to compare the amino acid sequences, for example, it is possible to use PAM120 weight residue table, gap length penalty 12, gap penalty 4. The amino acid sequence identity may be determined by any of the above methods, and upon calculation, the method of exhibiting the lowest value can be employed.

In the fusion protein, the Cryj1 mature protein may be present in the N terminal side and the Cryj2 mature protein may be present in the C terminal side, or Cryj1 mature protein may be present in the C terminal side and the Cryj2 mature protein may be present in the N terminal side. The fusion protein may or may not contain a peptide linker between the Cryj1 mature protein and the Cryj2 mature protein. Those skilled in the art can appropriately design the peptide linker by technical commonsense in the art. For example, the peptide linker can have a length of about 30 or less, preferably about 25 or less, more preferably about 20 or less, still more preferably about 15 or less and most preferably about 10 or 5 or less amino acid residues.

In the fusion protein, a further peptide moiety may be added to either the N terminus or the C terminus, or both. Such a peptide moiety is not particularly limited as long as it keeps the property of the fusion protein when added to the fusion protein. Such a peptide moiety includes, for example, tags for purification (e.g., histidine (His) tag, FLAG tag, Myc tag). Such a peptide moiety can have the length of about 30 or less, preferably about 25 or less and more preferably about 20 or less amino acid residues.

The fusion protein can be a soluble protein. When the fusion protein is the soluble protein obtained in a soluble fraction, there are merits in that the fusion protein can be purified with high purity by a general purification method such as column chromatography and can be easily modified. Even if the fusion protein is obtained in an insoluble fraction, it can be obtained as the soluble protein by a solubilization treatment, and thus also has the above merits.

An anaphylaxis reaction is caused by intracellular introduction of the signal produced by binding the allergen to an IgE antibody bound to the surface of mast cells. The fusion protein not only does not induce the production of the IgE antibody specific for the cedar pollen antigen (e.g., natural type Cryj1) but also can inhibit the production of the IgE antibody specific for the cedar pollen antigen by the cedar pollen antigen. Meanwhile, the fusion protein can hold one or more (preferably all) T cell epitopes in the Cryj1 mature protein and the Cryj2 mature protein. Therefore, the fusion protein has advantages in that the fusion protein can be the safe allergen incapable of causing the anaphylaxis reaction, it is possible to sufficiently induce the immunity (e.g., cellular immunity, humoral immunity such as IgG) specific for the cedar pollen antigen, capable of suppressing the degree of the anaphylaxis reaction caused by the cedar pollen and the T cell epitopes can be covered in all patients with cedar pollen disease, because the fusion protein can not be bound to the IgE antibody specific for the natural type Cryj1 or Cryj2.

### [Application to autoimmune diseases]

The autoantigen is not particularly limited as long as it is the antigen capable of being targeted by immune cells in the autoimmune disease. The autoantigen includes, for example, collagen, nucleic acids (Rheumatoid arthritis, systemic lupus erythematosus), myelin basic protein (multiple sclerosis), thyroglobulin (thyroid autoimmune disease), and graft alloantigen (graft versus host disease).

The pharmaceutical of present invention is useful as the therapeutic agent for the autoimmune disease when the pharmaceutical comprises the drug delivery vehicle comprising the autoantigen as the target antigen. The present inventors have found that the drug delivery vehicle comprising the CD1d ligand and the allergen can treat specifically the allergic disease caused by the allergen, and thus have conceived that the autoimmune disease caused by the autoantigen can be likewise treated by taking advantage of the drug delivery vehicle comprising the autoantigen instead of the allergen. Such an autoimmune disease includes, for example those described above.

An individual to which the drug delivery vehicle of the present invention can be administered can be any animal species. Such an animal species includes, for example, mammalian animals such as primates and rodents, and birds. More particularly, for example, human beings, monkeys, chimpanzees, dogs, cats, horses, cattle, swines, goats, sheeps, mice, rats, guinea pigs, hamsters, rabbits and chickens are included. In terms of clinical application, human beings, and/or dogs and cats are preferable.

The pharmaceutical of the present invention can comprise an optional carrier, e.g., a pharmaceutically acceptable carrier in addition to the drug delivery vehicle. The pharmaceutically acceptable carrier includes, but is not limited to, for example, excipients such as sucrose, starch, mannit, sorbit, lactose, glucose, cellulose, talc, calcium phosphate and calcium carbonate; binders such as cellulose, methylcellulose, hydroxypropylcellulose, gelatin, gum arabic, polyethylene glycol, sucrose and starch; disintegrants such as starch, carboxymethylcellulose, hydroxypropyl starch, sodium-glycol-starch, sodium hydrogen carbonate, calcium phosphate and calcium citrate; lubricants such as magnesium stearate, aerosyl, talc and sodium lauryl sulfate; aromatic substances such as citric acid, menthol, glycyl lysine ammonium salts, glycine and orange powder; preservatives such as sodium benzoate, sodium hydrogen sulfite, methylparaben and propylparaben; stabilizers such as citric acid, sodium citrate and acetic acid; suspending agents such as methylcellulose, polyvinyl pyrrolidone and aluminium stearate; dispersants such as surfactants; diluting agents such as water, saline and orange juice; and base waxes such as cacao butter, polyethylene glycol and illuminating kerosine.

Formulations suitable for oral administration are liquid agents dissolving an effective amount of the substance in the diluting agent such as water and saline; capsule agents, sachet agents and tablets containing the effective amount of the substance as a solid or a granule; suspension liquid agents suspending the effective amount of the substance in an appropriate dispersion medium; emulsions dispersing the solution in which the effective amount of the substance has been dissolved in the appropriate dispersion medium, or powders and granules.

As the formulations suitable for parenteral administration (e.g., intravenous injection, subcutaneous injection, intramuscular injection, topical injection), aqueous or non-aqueous isotonic sterile injectable liquid agents are available, and antioxidants, buffers, bacteriostats and tonicity agents may be contained therein. The formulation also includes aqueous and non-aqueous sterile suspension agents, and suspending agents, solubilizing agents, thickeners, stabilizers and preservatives may be contained therein. The formulation can be enclosed in a vessel such as an ampoule and a vial for a unit dosage or multiple dosages. The active component and the pharmaceutically acceptable carrier can also be lyophilized and stored for dissolving or suspending in an appropriate sterile vehicle just before the use.

A pharmaceutically effective amount of the agent of the present invention varies depending on an activity and a type of the active component, a dosing mode (e.g., oral, parenteral), severity of the disease, an animal species subjected to the administration, drug acceptability, body weight and age of a subject to be administered, and thus can not be flatly determined, but is typically about 0.1 to about 100 mg per day per kg body weight as the active component amount for an adult. The agent of the present invention may be administered to the subject (particularly human patient) having the immune disease consecutively for one to several days or with an interval of one to several days. For example, when the immune disease is the pollen disease, the agent of the present invention can be administered to the subject with pollen disease before or during the dispersal of the pollen (e.g., of the cedar, ragweed or the like) to be subjected. In the case of the other allergic diseases, it is desirable to administer before the subject is contacted with the allergen or when the allergy symptom appears after being contacted with the allergen.

The present invention will be described more specifically with reference to the following Examples, but these are merely for exemplification and do not limit the scope of the present invention.

### EXAMPLES

### Production Example 1: Production of liposome containing α-galactosyl ceramide and natural type Cryj1 protein

L-α-Phosphatidylglycerol, dipalmitoyl (DPPG, 1.12 mg, Wako Pure Chemical Industries Ltd.), 0.029 mg of 1,2-distearoyl-sn-glycero-3-phosphoethanolamine-N-[methoxy(polyethylene glycol)-2000] (Ammonium Salt) (PEG-PE; Avanti Polar Lipids) were dissolved in 250 µL of chloroform/methanol (1:1) solvent. Separately, 0.16 mg of α-galactosyl ceramide (made at RIKEN Research Center for Allergy and immunology) was dissolved in 250 µL of chloroform/methanol (1:1) solvent. Subsequently, both were mixed and dried in an evaporator, and dried overnight in a desiccator under vacuum. Then, 200 µL of an aqueous solution containing a Cryj1 protein (Seikagaku Kogyo Co., Ltd.) at a concentration of 0.4 mg/mL purified from natural cedar pollens was added. The mixture was treated using an ultrasonic pulverizer for 10 minutes and passed through a membrane having a pore size of 0.22 µm for sterilization. Subsequently, particles were sorted by passing 25 times through LiposoFast-Basic extruder (Avestin Inc.) loaded with a polycarbonate membrane having the pore size of 100 nm. The Cryj1 protein which had not been enclosed in the liposome was removed by concentrating the liposome enclosing Cryj1 using Amicon Ultra-4 centrifugation filter (PL-100) (Millipore) and washing with purified water to finally adjust 800 µL of the aqueous solution using the purified water. This aqueous solution containing the liposome enclosing the natural type Cryj1 (αGC-natural type Cryj1 liposome) was analyzed on SDS electrophoresis. As a result, it was identified that the concentration of the Cryj1 protein was 50 µg/mL. Supposing that all α-GalCer had been incorporated into the liposome membrane, and the final concentration of α-GalCer in the Lipo-αGC+Cryj1 solution was rendered 200 µg/mL.

### Example 1: Induction of IL-10 production from B220⁺ cells by liposome containing α-galactosyl ceramide

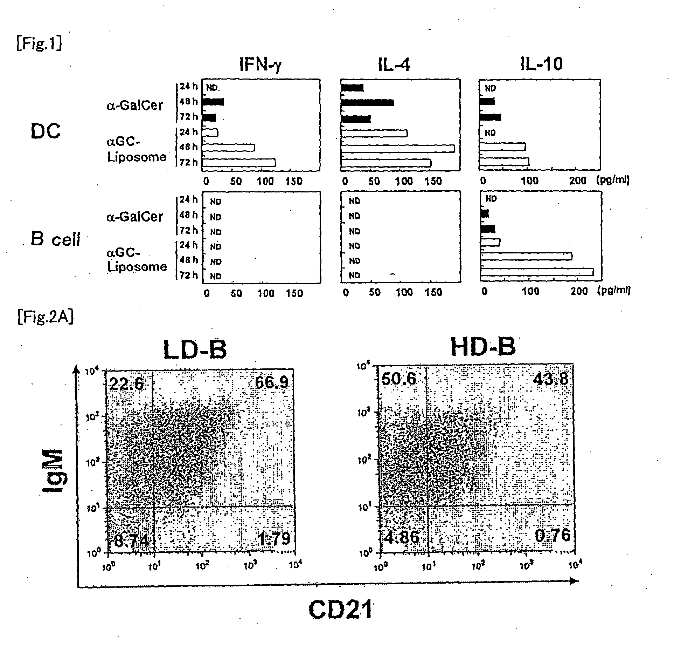

Aqueous α-GalCer or αGC liposome (International Publication WO2005/120574) at 2 µg α-GalCer/mouse was intraperitoneally administered to BDF1 mice, and after 24 hours, spleen was removed. The spleen was homogenized with a slide glass to prepare a cell suspension. Subsequently, anti-CD11c antibody magnetic beads (Miltenyi) were added thereto and CD11c⁺ cells (DC) were prepared using a magnet. B220⁺ cells (B cells) were prepared using anti-B220 mAb magnetic beads (Miltenyi) from the remaining cells which had not been bound to the magnet. Then, 2.5 x 10⁵ whole spleen cells from the normal BDF1 mouse and 1 x 10⁵ DC or 3 x 10⁵ B cells derived from the spleen in the BDF1 mouse administered with aqueous α-GalCer or αGC liposome, suspended in 200 µL of culture medium were added in one well in a 96-well U bottom culture plate, and cultured in an incubator containing 5% CO₂ at 37°C. Levels of cytokines, IFN-γ, IL-4 and IL-10 in culture supernatants after 24, 48 and 72 hours were measured by ELISA (FIG. 1).

As a result, in the group of adding CD11c⁺ cells derived from the spleen in the BDF1 mouse administered with αGC liposome, the amount of IFN-γ production was larger but the amounts of IL-4 and IL-10 production were equivalent compared with those in the group of adding CD11c⁺ cells derived from the spleen in the BDF1 mouse administered with aqueous α-GalCer. When B220⁺ cells derived from the spleen in the BDF1 mouse administered with αGC liposome were added to the whole spleen cells, IL-10 was produced but IL-4 and IFN-γ were not produced. When B220⁺ cells derived from the spleen in the BDF1 mouse administered with aqueous α-GalCer were added to the whole spleen cells, the production of IL-4, IL-10 and IFN-γ was not detected.

### Example 2: Induction of IL-10 production from marginal zone B cells by liposome containing α-galactosyl ceramide

αGC liposome at 2 µg α-GalCer/mouse was intraperitoneally administered to BDF1 mice, and after 24 hours, spleen was removed. Subsequently, 1 mg/mL collagenase D (Roche) was injected in the spleen, and the spleen was incubated in the CO₂ incubator for 45 minutes. Cells were extracted from the spleen, were suspended in 3 mL of HistoDenz (14.1%, Sigma-Aldrich), and X-VIVO 15 medium containing 50 µM 2-mercaptoethanol (2ME) (CAMBREX Bio Science Walkersville, Inc.) was overlaid. After centrifuging at 1500 rpm for 5 minutes, low density (LD) cells at an intermediate layer and precipitated high density (HD) cells were collected. The cells were washed with X-VIVO 15 medium containing 50 µM 2ME and 10% FCS, and suspended in phosphate buffered saline (PBS) containing 0.5% FCS. The anti-CD11c mAb magnetic beads (Miltenyi) were added to the LD cells, the CD11c⁺ dendritic cells were prepared using the magnet, and subsequently, LD-B cells were prepared using the anti-B220 mAb magnetic beads (Miltenyi) from the remaining cells. HD-B cells were also prepared using the anti-B220 mAb magnetic beads (Miltenyi) from the HD cells. The LD-B cells and the HD-B cells were stained with FITC-labeled anti-IgE antibody and PE-labeled anti-CD21 antibody, and subsequently analyzed by a flow cytometer (FIG. 2A). Subsequently, 2.5 x 10⁵ whole spleen cells from the normal BDF1 mouse and 1 x 10⁵ LD-B cells or HD-B cells, suspended in 200 µL of the culture medium were added to one well of a 96-well culture plate, and cultured in the incubator containing 5% CO₂ at 37°C.

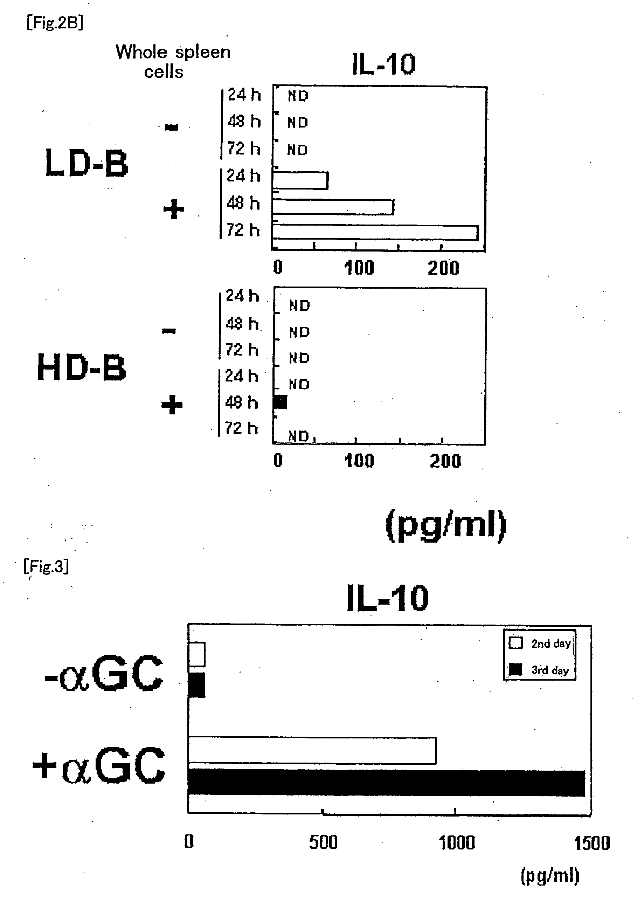

As a result, only in the group of adding the LD-B cells to the whole spleen cells, the IL-10 production was detected in the culture supernatant (FIG. 2B). The LD-B cell is a marginal zone B cell. Thus, it was shown that the IL-10 production was induced by interacting the marginal zone B cells obtained by *in vivo* administration of αGC liposome with the whole spleen cells.

### Example 3: Induction of IL-10 production from marginal zone B cells pulsed with liposome containing α-galactosyl ceramide

Into the spleen removed from the BDF1 mouse, 1 mg/mL of collagenase D (Roche) was injected, and the spleen was incubated in the CO₂ incubator for 45 minutes. Cells were collected from the spleen, and suspended in 3 mL of HistoDenz (14.1%, Sigma-Aldrich). Subsequently, X-VIVO 15 medium (CAMBREX Bio Science Walkersville, Inc.) containing 50 µM 2-mercaptoethanol (2ME) was overlaid. After centrifuging at 1500 rpm for 5 minutes, the low density (LD) cells at the intermediate layer were collected. The LD cells were washed with X-VIVO 15 medium containing 50 µM 2ME and 10% FCS, and suspended in phosphate buffered saline (PBS) containing 0.5% FCS. The anti-CD11c mAb magnetic beads (Miltenyi) were added to the LD cells to prepare CD11c⁺ dendritic cells, and subsequently, LD-B cells were prepared from the remaining cells using the anti-B220 mAb magnetic beads. Then, 3 x 10⁶ LD-B cells were added to a well of a 6-well culture plate, in which 3 mL of the culture medium had been placed. Further, αGC-liposome at a final concentration of 100 ng/mL was added to the culture medium in the well, or was not added. After culturing in the incubator containing 5% CO₂ at 37°C, the cells were collected from each well. Whole spleen cells (- B220 positive cells) were prepared by adding the anti-B220 mAb magnetic beads (Miltenyi) to the whole spleen cells derived from the BDF1 mouse and removing B220⁺ cells using the magnet. Subsequently, 2.5 x 10⁵ whole spleen cells (-B220 positive cells) from the normal BDF1 mouse and 1 x 10⁵ LD-B cells suspended in 200 µL of the culture medium were added into one well of the 96-well U bottom culture plate, and cultured in the incubator containing 5% CO₂ at 37°C for 2 or 3 days.

As a result, only in the group of adding LD-B cells cultured with αGC-liposome to the whole spleen cells (- B220 positive cells), the IL-10 production was detected in the culture supernatant (FIG. 3). The LD-B cell is the marginal zone B cell. Thus, it was shown that the marginal zone B cells pulsed with αGC-liposome induced the IL-10 production by interacting with the whole spleen cells.

### Example 4: Inhibition of in vivo secondary IgE antibody production by administration of liposome containing α-galactosyl ceramide and natural type Cryj1 protein

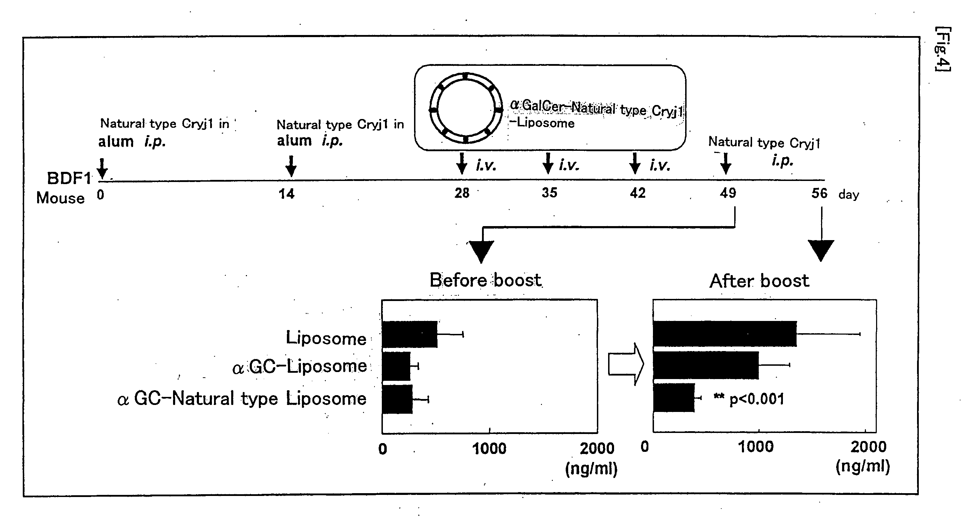

αGC-natural type Cryj1-liposome (αGC: 2 µg, Cryj1: 0.5 µg/mouse), αGC-liposome (αGC: 2 µg/mouse) or the liposome alone was intravenously administered three times to BDF1 mice sensitized twice with the natural type Cryj1 (0.5 µg/mouse, Seikagaku Kogyo Co., Ltd.) and aluminium hydroxide gel (2 mg/mouse) on the 28th, 35th and 42nd day after the sensitization. Boost immunization with the natural type Cryj1 (1 µg/mouse) was given on the 49th day. Blood samples were collected before and after the boost immunization, and levels of the anti-Cryj1 IgE antibody in serum were measured. As a result, in the group of administering αGC-natural type Cryj1-liposome, the secondary IgE antibody production after the boost immunization was significantly inhibited (FIG. 4).

### Example 5: Synthesis of Cryj1/2 fusion gene

The Cryj1/2 fusion gene was made by ligating the nucleotides (mature Cryj1) from 63rd (Ser) to 1122nd (Cys) containing no N terminal signal region in the Cryj1 gene (GenBank accession number: BAA07020) to the nucleotides (mature Cryj2) from 138th (Arg) to 1299th (Ser) containing no N terminal signal region in the Cryj2 gene (GenBank accession number: P43212) by the following methods. In the mature Cryj2 gene region, a XbaI cleavage sequence is present as the nucleotide sequence (TCTAGA) at positions 868 to 874 (Ser to Arg). It is inconvenient for recombination manipulation with various vectors, and thus, the XbaI cleavage sequence was deleted by codon substitution of TCTAGA→TCAAGA. First, 20 cycles of PCR using a plasmid DNA (Forestry and Forest products Research Institute) in which the full length Cryj2 gene had been inserted as a template and using primers 1 and 2 or primers 3 and 4 were performed in the presence of DNA polymerase (e.g., PrimeStar from Takara Shuzo Co., Ltd., or KOD from Toyobo Co., Ltd.) with high accuracy for DNA synthesis. As a result, an N terminal region fragment and a C terminal region fragment of the mature Cryj2 could be amplified. Subsequently, the mature Cryj2 gene in full length was amplified by mixing these two DNA fragments and performing 20 cycles of PCR using the primers 1 and 4. This DNA fragment was subcloned into XbaI-EcoRI site of the vector pMAT324, then DNA sequencing was performed and it was confirmed that the XbaI cleavage sequence had been deleted and no mutation due to PCR had occurred in the mature Cryj2 gene (pMAT324-Cry j2ΔXbaI). In order to make the Cryj1/2 fusion gene, the mature Cryj2 gene was amplified by PCR with pMAT324-Cry j2ΔXbaI as the template using the primers 4 and 5. Subsequently, the mature Cryj1 gene was amplified by PCR with the plasmid DNA (Forestry and Forest products Research institute) in which the full length Cryj1 gene had been inserted as the template using the primers 6 and 7. Finally, a Cryj1/2 fusion gene DNA fragment was amplified by mixing a mature Cryj1 gene fragment with a mature Cryj2 gene fragment and performing 20 cycles of PCR using the primers 4 and 6. The Cryj1/2 fusion gene was digested with EcoRI, and ligated to pET47b (Novagen) vector cleaved with SmaI and EcoRI to transform *Escherichia coli* DH10B strain (pET47b-Cryj1/2). The entire sequence of the Cryj1/2 fusion gene and a Histidine (His) tag sequence added at 5' terminus of the Cryj1/2 gene were confirmed by DNA sequencing (FIGs. 5 and 6).

Hereinafter, if necessary, the construct made in the present Example is abbreviated as recCryj1/2.
Primer 1 (sense): CCGGTCTAGAAAAGTTGAGCATTC (SEQ ID NO:1)
Primer 2 (antisense): CCTCTGCTCTTGAGTTTTCCC (SEQ 10 NO:2)
Primer 3 (sense): GGGAAAACTCAAGAGCAGAGG (SEQ ID NO:3)
Primer 4 (antisense): CCGGAATTCCTATCAACTTGGACTTAAATTC (SEQ ID NO:4)
Primer 5 (sense): AGAAAAGTTGAGCATTC (SEQ ID NO:5)
Primer 6 (sense): TCTGATAATCCCATAGAC (SEQ ID NO:6)
Primer 7 (antisense): GAATGCTCAACTTTTCTACAACGTTTAGAGAGAGAGC (SEQ ID NO:7)

### Example 6: Expression of recCryj1/2 protein

*Escherichia coli* BL21 strain (Invitrogen) was transformed with pET47b-Cryj1/2 plasmid DNA. The transformed strain was inoculated in 100 mL of LB medium containing kanamycin (final concentration: 20 µg/mL). The transformant was cultured at 37°C for 24 hours. Subsequently, 100 mL of the culture medium including the transformant was transferred to 1 L of the LB medium containing kanamycin, and the transformant was further cultured at 37°C for 2 hours. Then IPTG at a final concentration of 0.1 mM was added and the culture was continued at 30°C for 3 hours. Microbial cells were collected, and disrupted with ultrasound. A pellet was separated using a high speed centrifuge. The pellet suspended in water was analyzed together with a supernatant after the centrifugation by western blotting using SDS polyacrylamide gel electrophoresis and HRP-labeled anti-Cryj2 monoclonal antibody (Hayashibara Biochemical Laboratories Inc.). As a result, in a pellet fraction after disrupting the microbial cells, a protein which was positive for Coomassie brilliant blue (CBB) protein staining and positive for the anti-Cryj2 monoclonal antibody and had a molecular weight of about 70 kDa was predicted to be the recCryj1/2 protein. Subsequently, a band around 75 kDa transferred onto a PVDF membrane by western blotting was cut out after CBB staining, digested with an enzyme, endoprotease Asp-N (Takara Bio), and mass spectrometry was performed. As a result, many peptide sequences in Cryj1 and Cryj2 were detected.

From the above result, the protein of about 75 kDa in the pellet after disrupting the microbial cells was identified to be the recCryj1/2 protein.

### Example 7: Solubilization and purification of recCryj1/2 protein

pET47b-Cryj1/2 expression microbial cells (1 g, wet weight) was dissolved in 5 mL of Bugbuster (Novagen) and 1 µL of Benzonase (Novagen), which was then centrifuged at 16000 g for 20 minutes to collect an insoluble fraction. Then, 5 mL of Bugbuster (Novagen) was added thereto, and the reaction was thoroughly agitated by vortex, subsequently 2 µL Lysonase (Novagen) was added thereto, and the reaction was further agitated by vortex. After leaving stand at room temperature for 5 minutes, 30 mL of Bugbuster (Novagen) solution diluted 10 times was added thereto, and centrifuged at 16000 rpm for 15 minutes to collect an insoluble fraction. The pellet was suspended in 35 mL of 10 mM imidazole/8 M urea/phosphate buffered saline (PBS), and stirred using a magnetic stirrer to dissolve the insoluble protein. This solution was centrifuged at 18000 rpm for 15 minutes using the high speed centrifuge, and then the supernatant was collected. The centrifuged supernatant was applied using high performance liquid chromatography to a Chelating Sepharose FF column (GL Health Care Bioscience) filled with 0.1 M NiSO₄ and equilibrated with 50 mM imidazole/8 M urea/PBS. The column was washed with 50 mM imidazole/8 M urea/PBS, and then the recCryj1/2 fusion protein was eluted with 500 mM imidazole/8 M urea/PBS. The soluble recCryj1/2 protein was collected by adding arginine at a final concentration of 0.4 M to the eluate, placing the eluate in a dialysis tube and dialyzing in 0.4 M arginine/PBS solution for 24 hours. The collected protein was identified to be the recCryj1/2 protein by western blotting using the SDS polyacrylamide gel electrophoresis and anti-histidine monoclonal antibody (GE Health Care Bioscience) or HRP-labeled anti-Cryj2 monoclonal antibody (Hayashibara Biochemical Laboratories Inc.).

### Example 8: In vivo antibody production by immunization with recCryj1/2 protein

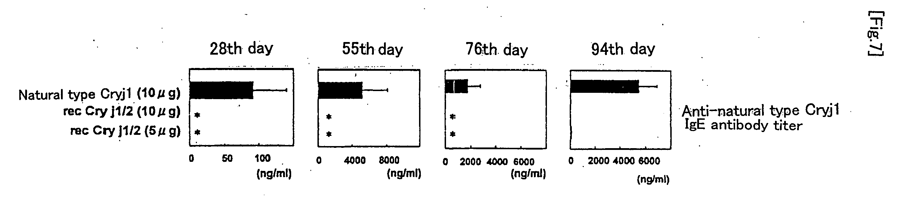

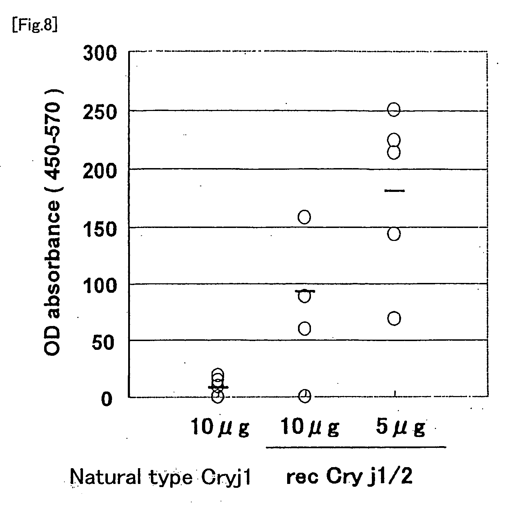

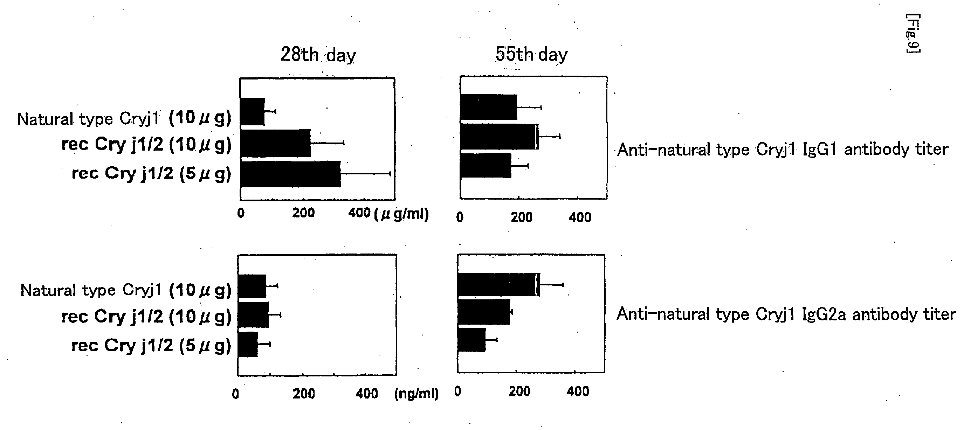

BALB/c x DBA/2F1 (BDF1) mice (female, 8 weeks of age, five mice in one group, Charles River) were intraperitoneally immunized with 10 µg of purified Cryj1 (natural type Cryj1, Hayashibara Biochemical Laboratories Inc.) derived from the cedar pollen, or 5 µg or 10 µg of the recCryj1/2 protein mixed with 2 mg of aluminium hydroxide gel adjuvant (RIKEN) at the start of the experiment (0day) and on the 14th day. The boost immunization with 1 µg of natural type Cryj1 or 5 µg or 10 µg of the recCryj1/2 protein was given to each mouse on the 41st day. Furthermore, on the 81st day, the boost immunization with 1 µg of natural type Cryj1 mixed with 2 mg of aluminium hydroxide gel was given to all mice. Blood samples were collected from orbital venous plexus on the 13th, 28th, 55th, 76th and 95th days, and levels of natural type Cryj1-specific antibody titers and recCryj1/2-specific IgE antibody titers were measured. As a result, the levels of the natural type Cryj1-specific IgE antibody titers on the 28th, 55th and 76th were increased in the mice immunized with natural type Cryj1, but they were not increased at all and the increase of the natural type Cryj1-specific IgE antibody on the 94th day against the immunization with aluminium hydroxide gel adjuvant and natural type Cryj1 on the 81st day was scarcely observed in the mice immunized with the recCryj1/2 protein (FIG. 7). The recCryj1/2-specific IgE antibody titers on the 76th day were increased in mice immunized with the recCryj1/2 fusion protein (FIG. 8). Meanwhile, on the 28th and 55th day, natural type Cryj1-specific IgG1 and IgG2a antibody titers were increased in both mice immunized with natural type Cryj1 and recCryj1/2 (FIG. 9).

From the above results, it is suggested that the recCryj1/2 protein could be a safe hyposensitization antigen which does not induce the production of the natural type Cryj1-specific IgE antibody.

### Example 9: Inhibitory capacity of recCryj1/2 protein for in vivo IgE antibody production

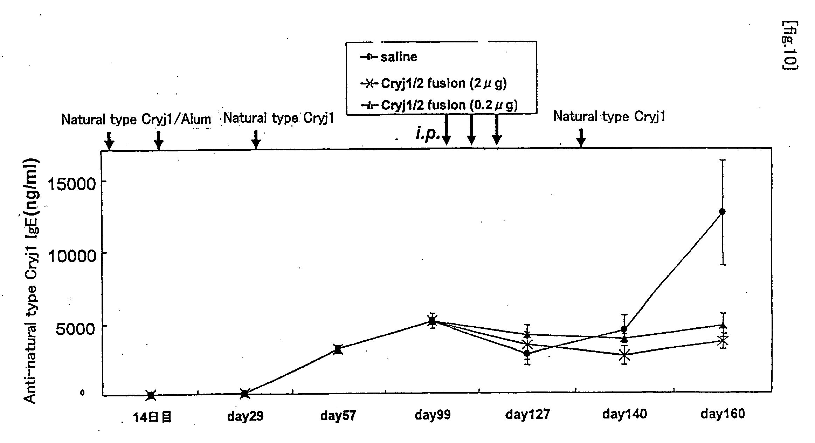

BDF1 mice (female, 8 weeks of age, Charles River) were intraperitoneally immunized with 5 µg of purified Cryj1 (natural type Cryj1, Hayashibara Biochemical Laboratories Inc.) derived from the cedar pollen mixed with 2 mg of aluminium hydroxide gel adjuvant (RIKEN) at the start of the experiment (0day) and on the 14th day. On the 50th day, the boost immunization with 1 µg of natural type Cryj1 was given. Meanwhile, the blood samples were collected from the orbital venous plexus on the 14th, 29th and 57th days, and the levels of the natural type Cryj1-specific IgE antibody in serum were measured. Subsequently, on the 99th day, the levels of the natural type Cryj1-specific IgE antibody were measured in all mice, and the mice were divided into three group (5 mice in one group) so that average values of antibody titers were equal among the groups. On the 106th, 113th and 120th days, the recCryj1/2 protein (0.2 µg), the recCryj1/2 protein (2 µg), or saline was administered three times, and further the boost immunization with 1 µg of natural type Cryj1 was given on the 133rd day. The blood samples were collected from the orbital venous plexus on the 140th and 160th days, and the levels of the natural type Cryj1-specific IgE antibody in serum were measured. As a result, in the group of administering the recCryj1/2, the natural type Cryj1-specific IgE antibody titers (the 127th day) was higher than those in the group of administering saline, but the subsequent natural type Cryj1-specific IgE antibody titers (the 160th day) after the boost immunization (the 133rd day) was increased in the group of administering saline, but conversely decreased in the group of administering the recCryj1/2 (FIG. 10),

From the above results, it is suggested that the recCryj1/2 protein could be utilized as the hyposensitization antigen which could inhibit the increase of the natural type Cryj1-specific IgE antibody titer.

### Production Example 2: Production of liposome containing α-galactosyl ceramide and recCryj1/2 (recombinant fusion protein)

L-α-Phosphatidylcholine, dioleoyl (DOPC, 0.77 mg, Wako Pure Chemical Industries Ltd.), 0.83 mg of cholesteryl 3β-N-(dimethylaminoethyl)carbonate hydrochloride (DC-Chol; Sigma-Aldrich) and 0.029 mg of 1,2-distearoyl-sn-glycero-3-phosphoethanolamine-N-[methoxy(polyethylene glycol)-2000] (ammonium salt) (PEG-PE; Avanti Polar Lipids) were dissolved in 250 µL of chloroform/methanol (1:1) solvent. Separately, 0.16 mg of α-galactosyl ceramide (made at RIKEN Research Center for Allergy and immunology) was dissolved in 250 µL of chloroform/methanol (1:1) solvent. Subsequently, both were mixed and dried in the evaporator, and dried overnight in the desiccator under vacuum. Then, 200 µL of an aqueous solution containing the recCryj1/2 protein (Example 7) at a concentration of 0.4 mg/mL was added. The mixture was treated using the ultrasonic pulverizer for 10 minutes and passed through the membrane having the pore size of 0.22 µm for sterilization. Subsequently, particles were sorted by passing 25 times through LiposoFast-Basic extruder (Avestin Inc.) load with the polycarbonate membrane having the pore size of 100 nm. The recCryj1/2 protein which had not been enclosed in the liposome was removed by concentrating the liposome enclosing the recCryj1/2 using Amicon Ultra-4 centrifugation filter (PL-100) (Millipore) and washing with purified water to finally adjust 800 µL of the aqueous solution using the purified water. This aqueous solution containing the liposome enclosing the recCryj1/2 (αGC-recCryj1/2 liposome) was analyzed on SDS electrophoresis. As a result, it was identified that the concentration of the recCryj1/2 protein was 25 µg/mL. Supposing that all α-GalCer had been incorporated into the liposome membrane, and the final concentration of α-GalCer in the Lipo-αGC+Cryj1 solution was rendered 200 µg/mL.

### Example 10: Inhibition of in vivo tertiary IgE antibody production by administrating liposome containing α-galactosyl ceramide and recCryj1/2 (recombinant fusion protein)

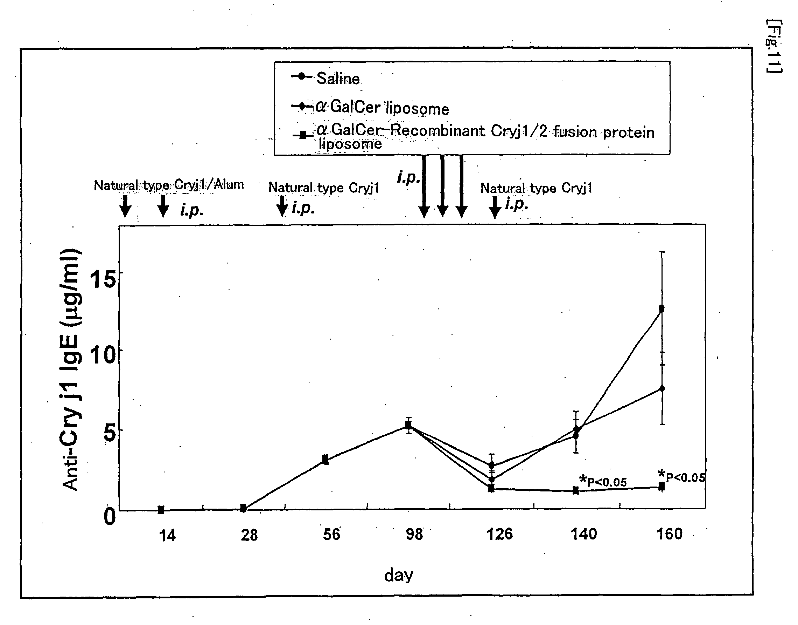

To BDF1 mice sensitized twice with the natural type Cryj1 (0.5 µg/mouse, Seikagaku Kogyo Co., Ltd.) and aluminium hydroxide gel (2 mg/mouse) followed by the boost immunization with the natural type Cryj1 (1 µg/mouse, Seikagaku Kogyo Co., Ltd.), αGC-recCryj1/2-liposome (αGC: 2 µg, recCryj1/2: 0.5 µg/mouse), αGC-liposome (αGC: 2 µg/mouse) or the liposome alone was intraperitoneally administered three times on the 105th, 112th and 119th days after the sensitization. The second boost immunization with the natural type Cryj1 (1 µg/mouse) was given on the 126th day. The blood samples were collected before the sensitization, and on the 14th, 28th, 56th, 98th, 126th, 140th and 160th days after the sensitization. The levels of the anti-Cryj1 IgE antibody in serum were measured by ELISA. As a result, in the group of administering αGC-liposome or αGC-recCryj1/2-liposome, the reduction of the IgE antibody titers in blood after the administration was notable and the increase of the tertiary IgE antibody production after the second boost immunization was also inhibited, compared with the negative control of administering the liposome alone (the inhibitory effect in the group of administering αGC-recCryj1/2-liposome was statistically significant) (FIG. 11). From the above results, it is suggested that the αGC-liposome could be anticipated to have the therapeutic effect of reducing the high IgE antibody titer after the occurrence of allergy, and that the effect could be further augmented by enclosing the antigen having no allergen property in the liposome.

### INDUSTRIAL APPLICABILITY

The agent of the present invention containing the drug delivery vehicle comprising the CD1d ligand and the target antigen (e.g., allergen, autoantigen) and having the lumen is useful for the treatment specific for the disease caused by the target antigen.

The agent of the present invention can comprise the fusion protein of the cedar pollen antigen. The fusion protein capable of being contained in the agent of the present invention has excellent effects in that the fusion protein can become the safe allergen which can not cause the anaphylaxis reaction, inhibit the degree of the anaphylaxis reaction caused by the cedar pollen, sufficiently induce the immunity specific for the cedar pollen antigen, and cover T cell epitopes in all patients with cedar pollen disease. Therefore, the agent of the present invention is useful in the novel hyposensitization therapy and/or as the pharmaceutical such as therapeutic vaccine.

The present application is based on JP-2006-005658 filed on January 13, 2006, and its content is incorporated herein by reference.

## Claims

1. A preventive or therapeutic agent for an immune disease caused by a target antigen, containing a drug delivery vehicle comprising a CD1d ligand and the target antigen and having a lumen.

2. The agent according to claim 1 wherein the target antigen is an allergen and the immune disease is an allergic disease caused by the allergen.

3. The agent according to claim 1 wherein the drug delivery vehicle is a liposome.

4. The agent according to claim 1 wherein the CD1d ligand is α-GalCer.

5. The agent according to claim 2 wherein the allergen is a cedar pollen.

6. The agent according to claim 2 wherein the allergen is a fusion protein of a Cryj1 mature protein and a Cryj2 mature protein.

7. The agent according to claim 5 wherein the Cryj1 mature protein is present in the N terminal side of the Cryj2 mature protein in the fusion protein.

8. The agent according to claim 2 wherein the CD1d ligand is embedded in a liposome membrane and the allergen is enclosed in a liposome lumen.

9. The agent according to claim 1 wherein the target antigen is an autoantigen.

10. A fusion protein comprising cedar pollen antigens, a Cryj1 protein and a Cryj2 protein.

11. The fusion protein according to claim 10 wherein said Cryj1 protein is a Cryj1 mature protein and the Cryj2 protein is a Cryj2 mature protein.

12. The fusion protein according to claim 10 wherein the Cryj1 protein and the Cryj2 protein are linked directly or via a peptide linker.

13. The fusion protein according to claim 10 wherein the Cryj1 protein is present in its N terminal side and the Cryj2 mature protein is present in its C terminal side.

14. A method for preventing or treating an immune disease comprising a step of administering a therapeutically effective amount of the preventive or therapeutic agent for the immune disease according to any of claims 1 to 9 to a subject in need of such a treatment.

15. The method for preventing or treating the immune disease according to claim 14 wherein said subject is a human patient with the immune disease.
